# EUROPEAN PATENT APPLICATION

(11) **EP 1 958 637 A1**
(43) Date of publication of application: **20.08.2008**
(21) Application number: 07102394.9
(22) Date of filing: 14.02.2007
(51) Int. Cl.: A61K 31/7004, A61P 31/12

(54) **Pharmaceutical composition for the treatment of IL-8 mediated diseases**

(71) Applicant: Revotar Biopharmaceuticals AG, 16761 Henningsdorf (DE)
(72) Inventor: Wolff, Gerhard, 16548, Glienicke-Nordbahn (DE); Meyer, Michael, 12209, Berlin (DE); Bock, Daniel, 30161, Hannover (DE); Aydt, Ewald M., 14163 Berlin (DE)
(74) Representative: Jacobi, Markus Alexander

(57) **Abstract**

A compound of formula (I) 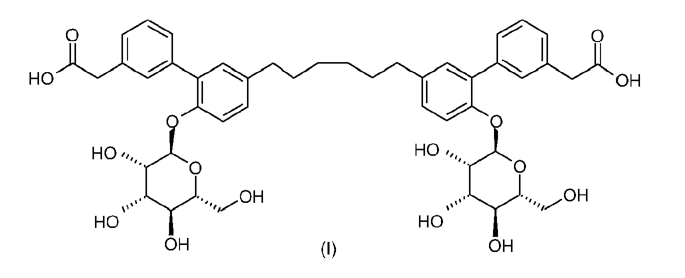 can be used for the preparation of a pharmaceutical composition for the treatment, diagnosis or prophylaxis of Interleukin-8 mediated disorders, e.g. various viral infections.

## Description

The present invention relates 1,6-Bis-[3-(3-carboxymethylphenyl)-4-(2-α-D-manno-pyranosyloxy)-phenyl] hexane and its use for the preparation of pharmaceutical compositions for the treatment of IL-8 mediated disorders.

Interleukin 8 (IL-8) is a chemokine normally produced by different cell types in humans, including macrophages, fibroblasts, endothelial cells, keratinocytes, melanocytes, chondrocytes and epithelial cells. In addition, IL-8 is also produced by injured cells and a variety of malignant cells and tumor cell lines. For IL-8 there exist historically a variety of alternative names or synonyms for IL-8 (e.g. Chemotaxin or NAP). IL-8 was renamed CXCL8 by The Chemokine Nomenclature Subcommittee of the Nomenclature Committee of the International Union of Immunological Societies, although its approved gene symbol remains IL8.

IL-8 is a non-glycosylated protein of 8 kDa (72 amino acids). Its gene SCYB8 mapping to human chromosome 4q12-q21 has a length of 5.1 kb and contains four exons. The 5' flanking region of the IL-8 gene contains potential binding sites for several nuclear factors including activation factor 1, activation factor-2, IFN regulator factor-1, a glucocorticoid responsive element, and a heat shock element. The mRNA of IL-8 consists of a 101 base 5' untranslated region, an open reading frame of 297 bases, and a long 3' untranslated region of 1.2 kb.

Upon stimulation, it is produced by processing of a precursor protein of 99 amino acids. Processing of this precursor by specific proteases yields N-terminal variants of IL-8. There exist also longer forms of IL-8 (79 and 77 amino acids) and shorter forms (69 amino acids) as well as a truncated variant of IL-8 lacking the first five amino acid identical with the fibroblast-derived neutrophil chemotactic factor (FDNCF). The predominant form of IL-8 is the 77 amino acid variant produced by endothelial cells.

In general, expression of IL-8 is not constitutive but can be stimulated by a variety of factors including Interleukin 1 (IL1) and Tumor Necrosis Factor-alpha (TNF-alpha) and co-stimulated by Interferon-Gamma (IFN-gamma). The synthesis of IL-8 is induced also by phytohemagglutinins, concanavalin A, double-stranded RNA, Phorbol esters, sodium urate crystals, viruses, and bacterial lipopolysacharids (LPS). There is also cell type specific stimulation of IL-8 expressions. For instance, the expression of IL-8 from resting and stimulated human blood monocytes is up-regulated by IL-7. In chondrocytes, the synthesis of IL-8 is stimulated by IL-1beta, TNF-alpha and LPS.

Upon processing, expressing cells release IL-8 to the local environment and systemic circulation where, soluble or immobilized to glycostructures e.g. glycosaminoglycans (GAGs), it may bind to members of the G-protein-coupled receptor protein family, named CXCR1 and CXCR2. CXCR1 and CXCR2 are expressed on the surface of many different cell types. The receptor density varies significantly between different cell types. For instance, in neutrophils it is approximately 20000/cell and in T-lymphocytes approximately 300/cell. CXCR2 also binds the chemokines CXCL1, CXCL2, CXCL3, CXCL5, CXCL6 and CXCL7 whereas only CXCL6 also binds CXCR1, but with a lower affinity than IL-8.

IL-8 is a cytokine mediating chemotactic activity against different leukocytes. In this context, after binding to its receptor IL-8 can induce a variety of biological reactions including transient increase in cytosolic calcium levels, the release of enzymes from granules and enhanced expression and avidity of adhesion molecules. IL8 is involved also in mediating pain. In addition, IL8 can antagonize IgE production by human B-cells without induced by IL4, affecting IgM, IgG1, IgG2, IgG3, IgG4, or IgA production as well as directly affects B-cells through a specific mechanism that is different from IFN-gamma, IFN-alpha, or prostaglandin E2. The intravenous administration of IL-8 in baboons causes a severe, albeit transient, granulocytopenia, followed by a granulocytosis, which persists as long as sufficient IL-8 levels are maintained. IL-8, has recently been shown to contribute to human cancer progression through its potential functions as a mitogenic, angiogenic, and motogenic factor.

IL-8 enhances the replication of several viruses including CMV, encephalomyocarditis virus (EMCV) and poliovirus. Others showed that viruses such as respiratory influenza virus, syncytial virus, and rotavirus induced IL-8 production. IL-8 may inhibit the antiviral action of IFN. The capacity of viruses to induce IL-8 in vitro and in vivo, the enhancement of viral replication by IL-8, and the interference with IFN- antiviral action against viruses may constitute a common strategy by which viruses take advantage of the host proteins for their own survival.

This is opposite to the IFN system, which is to protect cells from viruses, and also shown to inhibit IL-8 synthesis. The presence of IL-8 induced by viruses or other stimuli is supposed to the low potency of IFN in vivo during therapy and disease. Interference with IL-8 production or action may suppress viral activity and/or augment endogenous IFN- antiviral action against selected viruses. Also, intervention of IL-8 action or production may be useful as a mean of supplementing IFN- therapy and hence, enhancing of IFN- antiviral potency or reduction of its toxicity.

IL-8 is produced by various cancer cells, including human ovarian cancer cells, prostate cancer cells, and gastric cancer cells. IL-8 has been shown to enhance production and secretion of collagenase type IV by cancer cells, suggesting that it can modulate invasiveness and/or extracellular matrix remodeling in the cancer environment. As cell proliferation, angiogenesis, migration, and invasion are all important components of the metastatic process, IL-8 expression by tumor cells can influence their metastatic capabilities. Indeed, the expression of IL-8 has been shown to correlate with the metastatic potential of human melanoma cells, human ovarian cancer cells, human gastric carcinoma cells, and human prostate cancer cells. Anti-angiogenesis therapy involving the herein described invention may also be useful as an adjunct to chemotherapy, radiation therapy, or surgery in the treatment of angiogenic-related diseases.

Based on its pluri-potent functions, IL-8 is suggested to play a significant role in a wide variety of diseases and pathological stages including neurological disorders, cardiovascular disorders, hematological disorders, gastrointestinal and liver diseases, inflammation, diseases related to urology, metabolic disorders, and viral infections as well as cancer. In addition, IL-8 has been suggested to be used as biomarker for diseases and pathological stages. It has been shown that elevated levels of IL-8 correlates to a higher risk of getting diseases in the offspring, progression of human cancer as well as severity of a variety of diseases. In this respect, it has been proposed that one alternative to treat diseases like viral infections is lowering the level of IL-8 [Yang Y.J. et al., Eur. Resp. J. 2006, 27(1), 12-19; Khabar K.S., J. Exp. Med. 1997, 186, 7, 1077-1085; Khabar K.S. et al., Biochem. Biophys. Res. Commun. 1997 Jun 27;235(3), 774-778].

Despite the progress in biomedical research, there is a tremendous medical need for novel effective and safe drugs for the treatment, diagnosis, and/or prophylaxis of neurological disorders, cardiovascular disorders, hematological disorders, gastrointestinal and liver diseases, inflammation, diseases related to urology, metabolic disorders, and viral infections as well as cancer. One alternative target to develop such novel drugs is to lower the level of IL-8.

As shown in experimental animal disease models and human clinical studies, lowering IL-8 is paralleled by an improvement of the diseased condition [Mahler D.A. et al., Chest. 2004, 126(3), 926-934]. Therefore, therapeutical means lowering the level of IL-8 like monoclonal antibodies or synthetical molecules are highly desired.

Surprisingly, it has been found that the compound of formula (I) (1,6-Bis [3-(3-carboxymethylphenyl)-4-(2-α-D-mannopyranosyloxy)-phenyl] hexane) modulates the level of IL-8. A recent human clinical trial showed that the compound of formula (I) down-modulates the IL-8 level in a statistically significant and clinically relevant manner (see Example 1).

The compound of formula (I) and its preparation have been described before. It consists of two identical parts of a biphenyl substituted acetic acid which are connected by an alkyl chain. The biphenyl moieties are further substituted with mannose. The herein showed absolute configuration is the preferred one, but other stereoisomeric forms are also active as well as salts, in particular the disodium salt of the compound of formula (I).

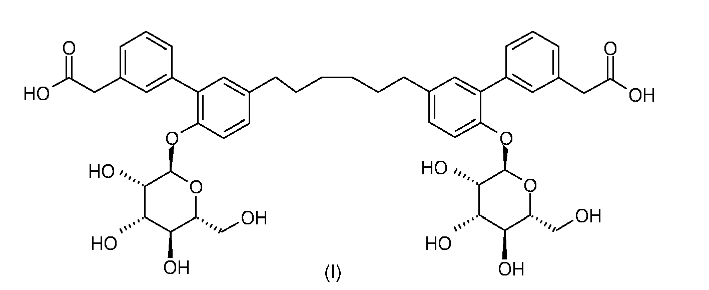

The compound of formula (I) occurs in different polymorphic or pseudo-polymorphic forms, whereby herein the term "FORM" is used to describe both polymorphs and pseudo-polymorphs including solvates and hydrates. The different FORMS, which can be prepared specifically by adjustment of the reaction conditions and/or of the crystallization conditions, differ in their structural and physicochemical properties as well as in their pharmacological properties, e.g. bioavailability.

The FORMS differ, for example, in the melting points, in their solubility, rate of dissolution or the behavior during pharmaceutical processing and allow the production of pharmaceutical compositions having different property profiles starting from a single parent compound.

FORM 1 of compound of formula (I) is characterized by a strong X-ray reflection angle 2Theta (in °) in the X-ray diffraction diagram using Cu Kα1 radiation at 2Theta = 4.8 .

FORM 2 of compound of formula (I) has X-ray reflections diffraction angles 2Theta (in °) in the X-ray diffraction diagram using Cu Kα1 radiation with strong intensity at 2Theta = 5.3 and 5.6 and 17.4° and with medium intensity at 2Theta = 15.1 °.

FORM 3 of compound of formula (I) has X-ray reflections diffraction angles 2Theta (in °) in the X-ray diffraction diagram using Cu Kα1 radiation with strong intensity at 2Theta = 5.3 and 5.6° and with medium intensity at 2Theta = 4.2, 4.3 and 4.8°

The compound of formula (I) has valuable pharmacological properties. It has been described previously as an antagonist of E-, P-, and L-selectins that inhibits the binding of sialyl-Lewis^{x} (sLe^{x}) and sialyl-Lewis^{a} (sLe^{a}) to the before mentioned vascular cell-adhesion proteins [WO97/01335; Kogan T.P. et al., J. Med. Chem. 1998, 41, 1099-1111]. Selectins mediate the initial rolling of leukocytes on the vascular endothelium following inflammation or injury. As leukocyte rolling is a key event in inflammatory response, selectins are considered as targets for the treatment of inflammation.

The compound of formula (I) is well tolerated and was described to be relevant for the treatment of diseases related to inflammation, cell-cell recognition and adhesion, for example acute lung injury (ALI), acute respiratory distress syndrome (ARDS), asthma, chronic obstructive pulmonary disease (COPD), psoriasis, rheumatoid arthritis, sepsis, Crohn's disease, traumatic shock, multi organ failure, autoimmune diseases, inflammatory bowel disease and reperfusion tissue injury associated with organ transplantation, heart attacks or strokes. As sLe^{a} is located on some cancer cells, cell adhesion involving sLe^{a} might be involved in the metastasis of certain cancers. Therefore compound (I) may be useful for the treatment of some forms of cancer such as lung or colon cancer.

However, the effect of the compound of formula (I) on the level of IL-8 and the use of the compound of formula (I) for the preparation of a pharmaceutical composition for the treatment, diagnosis, and prophylaxis of IL-8-mediated disorders and/or pathological conditions has not been described.

The surprising result of down-regulation of IL-8 levels by the administration of the compound of formula (I) and the impact of IL-8 on a variety of diseases and pathological conditions offer new opportunities for the treatment, diagnosis, and prophylaxis of medical indications with the pharmaceutical compositions comprising the compound of formula (I) and eventually further pharmaceutically inactive and/or pharmaceutically active ingredients.

The present invention relates to the use of the compound of formula (I) for the preparation of a pharmaceutical composition for the treatment, diagnosis, or prophylaxis of Interleukin-8 mediated disorders.

The term "disorders" is used herein to describe diseases, disorders and pathological conditions.

The term "compound of formula (I)" is used herein to describe the compound of formula (I) as shown in this invention, a salt of the compound of formula (I) or a stereoisomeric or polymorphic form thereof.

IL-8 plays a crucial role in the pathogenesis of viral infections. High levels of IL-8, which can also be induced by viruses, enhance the replication of viruses. High level of IL-8 can also inhibit the antiviral action of Interferon (IFN). Thus, lowering the level of IL-8 in the course of viral infections is highly desired.

The use of the compound of formula (I) for the preparation of various types of pharmaceutical compositions for the treatment, diagnosis, or prophylaxis of Interleukin-8 mediated disorders of viral infections is also subject of this invention.

A preferred embodiment of this invention is the use of the compound of formula (I) for the preparation of a pharmaceutical composition for the treatment, diagnosis, or prophylaxis of viral infections particularly caused by cytomegalovirus (CMV), encephalomyocarditis virus (EMCV), poliovirus, influenca virus, parainfluenza respiratory influenza virus, respiratory syncytial virus, Japanese encephalitis virus, Dengue virus, hepatitis A virus (HAV), hepatitis B virus (HBV), hepatitis C virus (HCV), hepatitis D virus (HDV), hepatitis E virus (HEV), hepatitis F virus (HFV), hepatitis G virus (HGV) Epstein Barr Virus (EBV), human immunodeficiency virus type 1 (HIV-1), human immunodeficiency virus type 2 (HIV-2), varicella zoster virus, herpes simplex virus, in particular herpes simplex virus type 1 (HSV-1), herpes simplex virus type 2 (HSV-2), or human herpes virus 1, 2, 3, 4, 7, or 8, Kaposi's sarcoma-associated herpesvirus (KSHV), rotavirus, papilloma virus, and human papilloma virus (HPV), in particular HPV of the types: 1, 2, 3, 4, 5, 8, 9, 11, 12, 13, 14, 15, 16, 17, 18, 19-29, 31, 32, 34, 36-38, 46-50, 56, or 58.

In particular viral skin diseases and/or tumor disorders can be treated with the compound of formula (I) such as genital warts, benign tumors of the skin and/or mucosa caused by papilloma viruses, in particular verrucae plantares, verrucae vulgares, verrucae planae juveniles, epidermodysplasia verruciformis, Condylomata acuminate, Condylomata plana, bowenoid papulosis, papilloma on the larynx and oral mucosa, focal epithelial hyperplasia, herpes labialis, varicella and shingles.

I1-8 is also involved in the pathogenesis of various disorders related to neurological disorders, including disorders of the central nervous system as well as disorders of the peripheral nervous system. The present invention relates to the use of the compound of formula (I) for the preparation of a pharmaceutical composition for the treatment, diagnosis, or prophylaxis of neurological disorders. Neurological disorders include, but are not limited to brain injuries, cerebrovascular disorders and their consequences, Parkinson's disease, corticobasal degeneration, motor neuron disease, multiple sclerosis, traumatic brain injury, stroke, post-stroke, post-traumatic brain injury, and small-vessel cerebrovascular disease, cognitive-related disorders such as mild cognitive impairment, age-associated memory impairment, age-related cognitive decline, vascular cognitive impairment, attention deficit disorders, attention deficit hyperactivity disorders, and memory disturbances in children with learning disabilities.

Pain, within the meaning of this definition, is considered to be a CNS disorder and can be caused, at least in part, by viral infections. A preferred embodiment of this invention is the use of the compound of formula (I) for the preparation of a pharmaceutical composition for the treatment, diagnosis, or prophylaxis of pain. Pain can be associated with CNS disorders, in very particular multiple sclerosis, spinal cord injury, sciatica, failed back surgery syndrome, traumatic brain injury, epilepsy, Parkinson's disease, post-stroke, and vascular lesions in the brain and spinal cord including infarct, hemorrhage, and vascular malformation. Non-central neuropathic pain includes post mastectomy pain, phantom feeling, reflex sympathetic dystrophy (RSD), trigeminal neuralgiaradioculopathy, post-surgical pain, HIV/AIDS related pain, cancer pain, metabolic neuropathies including diabetic neuropathy and vasculitic neuropathy secondary to connective tissue disease, paraneoplastic polyneuropathy associated with carcinoma of lung or leukemia or lymphoma or carcinoma of prostate, colon, or stomach, trigeminal neuralgia, cranial neuralgias, and post-herpetic neuralgia.

Pain can also be associated with peripheral nerve damage, central pain due to cerebral ischemia and chronic pain i. e., lumbago, back pain (low back pain), inflammatory and/or rheumatic pain, headache pain, headache pain associated with migrane disorders, migraine with aura, migraine without aura, and episodic and chronic tension-type headache, tension-type like headache, cluster headache, and chronic paroxysmal hemicrania. Visceral pain including pancreatits, intestinal cystitis, dysmenorrhea, irritable Bowel syndrome, Crohn's disease, biliary colic, ureteral colic, and pain syndromes of the pelvic cavity including vulvodynia, orchialgia, urethral syndrome and protatodynia are also CNS disorders. Also considered to be a disorder of the nervous system are acute pain, for example postoperative pain and pain after trauma.

Dementia is also related to neurological disorders and viral infections mediated by IL-8. The use of the compound of formula (I) for the preparation of a pharmaceutical composition for the treatment, diagnosis, or prophylaxis of dementia is also subject of this invention. Dementia includes, but is not limited to dementia including amyotrophic lateral sclerosis, Alzheimer's disease, vascular dementia, dementia with Lewy bodies, frontotemporal dementia and Parkinsonism linked to chromosome 17, frontotemporal dementias, including Pick's disease, progressive nuclear palsy, corticobasal degeneration, Huntington's disease, thalamic degeneration, Creutzfeld-Jakob dementia, HIV dementia, schizophrenia with dementia, and Korsakofs psychosis.

IL-8 mediated disorders cover a wide range of further medical indications. Thus, the use of the compound of formula (I) for the preparation of a pharmaceutical composition for the treatment, diagnosis, or prophylaxis of cardiovascular disorders, urology-related disorders, hematological disorders, metabolic disorders, angiogenetic-related disorders, hyperprolifertive disorders, and gastrointestinal and liver disorders is also subject of this invention.

Cardiovascular disorders include acute and chronic high-output and low-output pumping failures, right-sided or left-sided pumping failures, systolic or diastolic pumping failures, myocardial infarction (MI), ischemic diseases such as stable angina, unstable angina and asymptomatic ischemia, arrhythmias including atrial and ventricular tachyarrhythmias, atrial tachycardia, atrial flutter, atrial fibrillation, atrio-ventricular reentrant tachycardia, preexitation syndrome, ventricular tachycardia, ventricular flutter, ventricular fibrillation, as well as bradycardic forms of arrhythmias, hypotension, hypertensive vascular diseases such as primary as well as secondary arterial hypertension, renal, endocrine, and neurogenic, peripheral vascular diseases such as chronic peripheral arterial occlusive disease (PAOD), acute arterial thrombosis and embolism, Raynaud's phenomenon and venous disorders, atherosclerosis, and congestive heart failure.

Cardiovascular disorders also include cardiovascular diseases, disorders, and pathological conditions caused by disorders of lipid metabolism such as hyperlipidemia, obesity, a high-fat diet, lack of exercise, moderate to high alcohol consumption, cigarette smoking, poorly controlled diabetes, and an underactive thyroid gland, hereditary hyperlipidemias (type I hyperlipoproteinemia (familial hyperchylomicronemia), type II hyperlipoproteinemia (familial hypercholesterolemia), type III hyperlipoproteinemia, type IV hyperlipoproteinemia, or type V hyperlipoproteinemia), hypolipo- proteinemia, lipidoses (caused by abnormalities in the enzymes that metabolize fats), Gaucher's disease, Niemann-Pick disease, Fabry's disease, Wolman's disease, cerebrotendinous xanthomatosis, sitosterolemia, Refsum's disease, or Tay-Sachs disease.

Disorders related to urology include benign and malign disorders of the organs constituting the genitourinary system of female and male, renal diseases such as acute or chronic renal failure, immunologically mediated renal diseases in such as renal transplant rejection, lupus nephritis, immune complex renal diseases, glomerulopathies, nephritis, toxic nephropathy, obstructive uropathies such as benign prostatic hyperplasia (BPH), neurogenic bladder syndrome, urinary incontinence in particular urge-, stress-, or overflow incontinence, pelvic pain, and erectile dysfunction.

Hematological disorders include anemias such as anemias due to defective or deficient synthesis, deficient erythropoiesis, myeloproliferative disorders such as polycythemia vera, tumor-associated erythrocytosis, myelofibrosis, thrombocythemia, hemorrhagic disorders such as vasculitis, thrombocytopenia, heparin-induced thrombocytopenia, thrombotic thrombocytopenic purpura, hemolytic-uremic syndrome, hereditary and acquired disorders of platelet function, and hereditary coagulation disorders, Leucopenia such as neutropenia, lymphocytopenia, Eosinophilic disorders such as hypereosinophilia, and idiopathic hypereosinophilic syndrome, macroglobulinemia, and heavy chain diseases, idiopathic thrombocytopenic purpura, iron deficiency anemia, megaloblastic anemia (vitamin B12 deficiency), aplastic anemia, thalassemia, malignant lymphoma bone marrow invasion, malignant lymphoma skin invasion, hemolytic uremic syndrome, giant platelet disease, and disorders of the Spleen in the course of hematological disorders.

Metabolic disorders include metabolic diseases affecting biochemical processes and tissues ubiquitous all over the body in particular obesity, amyloidosis, disturbances of the amino acid metabolism such as branched chain disease, hyperaminoacidemia, hyperaminoaciduria, disturbances of the metabolism of urea, hyperammonemia, mucopolysaccharidoses such as Maroteaux-Lamy syndrom, storage diseases such as glycogen storage diseases and lipid storage diseases, glycogenosis diseases such as Cori's disease, malabsorption diseases such as intestinal carbohydrate malabsorption, oligosaccharidase deficiency such as maltase-, lactase-, sucrase-insufficiency, disorders of the metabolism of fructose, disorders of the metabolism of galactose, galactosaemia, disturbances of carbohydrate utilization such as diabetes, hypoglycemia, disturbances of pyruvate metabolism, hypolipidemia, hypolipoproteinemia, hyperlipidemia, hyperlipoproteinemia, carnitine or carnitine acyltransferase deficiency, disturbances of the porphyrin metabolism, porphyrias, disturbances of the purine metabolism, lysosomal diseases, metabolic diseases of nerves and nervous systems such as gangliosidoses, sphingolipidoses, sulfatidoses, leucodystrophies, and Lesch-Nyhan syndrome.

Metabolic disorders also include metabolic diseases affecting the bone such as osteoporosis, osteomalacia such as osteoporosis, osteopenia, osteogenesis imperfecta, osteopetrosis, osteonecrosis, Paget's disease of bone, and hypophosphatemia.

Metabolic disorders also include metabolic diseases affecting the nervous system such as cerebellar dysfunction, disturbances of brain metabolism, Huntington's chorea, Pick's disease, toxic encephalopathy, demyelinating neuropathies, and Guillain-Barre syndrome.

Metabolic disorders also include metabolic diseases affecting the endocrine system such as Sipple's syndrome, pituitary gland dysfunction and its effects on other endocrine glands, such as the thyroid, adrenals, ovaries, and testes, acromegaly, hyper- and hypothyroidism, euthyroid goiter, euthyroid sick syndrome, thyroiditis, and thyroid cancer, over- or underproduction of the adrenal steroid hormones, adrenogenital syndrome, Cushing's syndrome, Addison's disease of the adrenal cortex, Addison's pernicious anemia, primary and secondary aldosteronism, diabetes insipidus, carcinoid syndrome, disturbances caused by the dysfunction of the parathyroid glands, pancreatic islet cell dysfunction, diabetes, disturbances of the endocrine system of the female in particular estrogen deficiency, resistant ovary syndrome.

Metabolic disorders also include metabolic diseases affecting the muscle including the heart such as muscle weakness, myotonia, Duchenne's, muscular dystrophia, dystrophia myotonica of Steinert, mitochondrial myopathies such as disturbances of the catabolic metabolism in the muscle, carbohydrate and lipid storage myopathies, glycogenoses, myoglobinuria, malignant hyperthermia, polymyalgia rheumatica, dermatomyositis, primary myocardial disease, and cardiomyopathy.

Metabolic disorders also include metabolic diseases affecting the skin and nervous tissue, the urological system, and the homeostasis of body systems like water and electrolytes such as disorders of the ectoderm, neurofibromatosis, scleroderma and polyarteritis, Louis-Bar syndrome, von Hippel-Lindau disease, Sturge-Weber syndrome, tuberous sclerosis, amyloidosis, porphyria, sexual dysfunction of the male and female, confused states and seizures due to inappropriate secretion of antidiuretic hormone from the pituitary gland, Liddle's syndrome, Bartter's syndrome, Fanconi's syndrome, renal electrolyte wasting, and diabetes insipidus.

Angiogenic-related disorders include disorders such as cancers, tumors, metastasis, and neoplasms. In particular angiogenic-related disorders include, but are not limited to ovarian cancer, pancreatic cancer, breast cancer, colon carcinoma, rectum carcinoma, esophagus carcinoma, stomach carcinoma, pancreas carcinoma, liver carcinoma, gallbladder carcinoma, bile duct carcinoma, small intestine carcinoma, urinary tract carcinoma, kidney carcinoma, bladder carcinoma, urothelium carcinoma, female genital tract carcinoma, cervix carcinoma, choriocarcinoma, gestational trophoblastic disease, male genital tract carcinoma, prostate carcinoma, seminal vesicle carcinoma, testes carcinoma, germ cell carcinoma, endocrine gland carcinoma, thyroid carcinoma, adrenal carcinoma, pituitary gland carcinoma, skin carcinoma, hemangiomas, melanomas, sarcomas, bone and soft tissue sarcoma, Kaposi's sarcoma, tumors of the brain, tumors of the nerves, tumors of the eyes, tumors of the meninges, astrocytomas, gliomas, glioblastomas, retinoblastomas, neuromas, neuroblastomas, schwannomas and meningiomas. Furthermore they include solid tumors arising from hematopoeitic malignancies such as leukemias, chloromas, plasmacytomas, plaques and tumors of mycosis fungoides and cutaneaous T-cell lymphomas or leukemias, gastric ulcers, duodenal ulcers, and ductal adenocarcinoma, cystadenocarcinoma, islet cell tumors, insulinoma, gastrinoma, carcinoid tumors, glucagonoma, neoplasms of the bowel such as familial polyposis, adenocarcinoma, primary malignant lymphoma, carcinoid tumors, polyps, benign or malignant neoplasms of the liver, Leukemias such as acute myeloic leukemia, acute lymphoblastic leukemia, chronic myelocytic leukemia, chronic lymphocytic leukemia, and myelodysplastic syndrome, Lymphomas such as Hodgkin's disease, non-Hodgkin's lymphoma, Burkitt's lymphoma, and Plasma Cell Dyscrasias such as multiple myeloma.

The term "angiogenetic-related disorders" includes disorders and temporary states of disorders where angiogenesis had been part of patogenesis, is part of it or might become part of it.

Hyperprolifertive disorders include diabetic retinopathy, dysplasia, hyperplasia, metaplasia, leukoplasia, non-bullous congenital ichthyosiform erythroderma, autosomal dominant polycystic kidney disease, Morbus Ledderhose, neovascular age-related macular degeneration, sickle cell anemia, pseudoxanthoma elasticum, Paget's disease or restenosis e.g. after surgery, degeneration, sickle cell anemia, pseudoxanthoma elasticum, Paget's disease or restenosis, metaplasia of gastric tissues, neoplasms of the stomach, neoplasms of the exocrine or endocrine pancreas such as multiple endocrine neoplasia syndrome.

Gastrointestinal and liver disorders include disorders of the esophagus such as achalasia, vigoruos achalasia, dysphagia, cricopharyngeal incoordination, pre-esophageal dysphagia, diffuse esophageal spasm, globus sensation, Barrett's metaplasia, and gastroesophageal reflux, disorders of the stomach and duodenum such as functional dyspepsia, gastritis, stress gastritis, chronic erosive gastritis, atrophy of gastric glands, disorders of the pancreas such as acute or chronic pancreatitis, insufficiency of the exocrinic or endocrinic tissues of the pancreas such as steatorrhea, diabetes, Zollinger-Ellison syndrome, Vipoma syndrome, and malabsorption syndrome, and functional bowel disorders such as irritable bowel syndrome.

Gastrointestinal and liver disorders also include disorders of the bilirubin metabolism, jaundice, syndroms of Gilbert's, Crigler-Najjar, Dubin-Johnson and Rotor; intrahepatic cholestasis, hepatomegaly, portal hypertension, ascites, Budd-Chiari syndrome, portal-systemic encephalopathy, fatty liver, steatosis, Reye's syndrome, liver diseases due to alcohol, alcoholic hepatitis or cirrhosis, fibrosis and cirrhosis, fibrosis and cirrhosis of the liver due to inborn errors of metabolism or exogenous substances, storage diseases, syndromes of Gaucher's, Zellweger's, Wilson's - disease, acute or chronic hepatitis, viral hepatitis and its variants, viral infections of the liver, fungi, protozoa, helminths; drug induced disorders of the liver, chronic liver diseases such as primary sclerosing cholangitis,alphal-antitrypsin-deficiency, primary biliary cirrhosis, postoperative liver disorders such as postoperative intrahepatic cholestasis, hepatic granulomas, vascular liver disorders associated with systemic disease, and disturbance of liver metabolism in the new-born or prematurely born.

Further IL-8 mediated disorders include allergy, gout, acute pseudogout, allograft rejection, chronic transplant rejection, mononuclear-phagocyte dependent lung injury, idiopathic pulmonary fibrosis, acute chest syndrome in sickle cell disease, fibromyalgia, ostoarthropathy and thrombosis.

The compound of formula (I) can also be used in combination with further active pharmaceutical compounds or ingredients. The terms "pharmaceutically active compound" and "active pharmaceutical ingredient" are interchangeably used herein to describe drug substances with pharmaceutical action. Active pharmaceutical ingredients are distinct from carriers or pharmaceutically tolerable components or inactive pharmaceutical ingredients used for the sole purpose of drug formulation.

The invention also relates to pharmaceutical compositions comprising the compound of formula (I) in combination with least one further pharmaceutically active ingredient.

The pharmaceutical compositions comprising the compound of formula (I) in combination with at least one further pharmaceutically active ingredient can be used for the treatment, diagnosis, and prophylaxis of the herein mentioned viral infections, neurological disorders including pain and dementia, cardiovascular disorders, urology-related disorders, hematological disorders, metabolic disorders, angiogenetic-related disorders, hyperprolifertive disorders, and gastrointestinal and liver disorders. In addition, the pharmaceutical compositions comprising the compound of formula (I) in combination with at least one further active pharmaceutical ingredient can be used for the treatment, diagnosis, and prophylaxis of inflammatory disorder such as chronic obstructive pulmonary disease (COPD), acute lung injury (ALI), cardiopulmonary bypass, acute respiratory distress syndrome (ARDS), septic shock, sepsis, psoriasis, atopic dermatitis, and rheumatoid arthritis, and reperfusion injury that occurs following heart attacks, strokes, atherosclerosis, and organ transplants, traumatic shock, multi-organ failure, autoimmune diseases like multiple sclerosis, percutaneous transluminal angioplasty, asthma, and inflammatory bowel diseases (particularly Crohn's disease).

Further pharmaceutically active ingredients that can be used in combination with the compound of formula (I) are anti-viral agents.

The anti-viral agents may be used, but are not limited to anti-viral agents used for the treatment of herpes, influenza, hepatitis, HIV, or warts such as acyclovir, cidofovir, famciclovir, foscarnet, fomivirsen, ganciclovir, idoxuridine, penciclovir, trifluridine, valacyclovir, valganciclovir, resiquimod, amantadine, oseltamivir, rimantadine, zanamivir, peramivir, R-118958, adefovir dipivoxil, IFN-alpha, lamivudine, pegylated interferon alpha, entecavir, clevudine, emtricitabine, telbivudine, tenofovir, viramidine, BILN2061, NM283, ribavirin, imiquimod, maribavir, sICAM-1, pleconaril, brivudin, zidovudine, didanosine, stavudine, zalcitabine, lamivudine, abacavir, tenofovir, tenofovir-disoproxil, emtricitabine, nevirapine, efavirenz, delaviridine, saquinavir, indinavir, ritonavir, nelfinavir, amprenavir, lopinavir, atazanavir, fosamprenavir, enfuvirtide, or polyphenolic compounds with antioxidative properties, such as mixtures of phytochemicals, in particular a mixture of catechins produced from a partially purified water extract of green tea leaves of the tea plant, Camellia sinensis (e.g. Polyphenone^{®} E).

Further pharmaceutically active ingredients that can be used in combination with the compound of formula (I) are antibiotics. They are, but are not limited to aminoglycosides such as amikacin, amikin, gentamicin, garamycin, kanamycin, neomycin, netilmicin, streptomycin, and tobramycin, carbacephems such as loracarbef, carbapenems such as ertapenem, imipenem/cilastatin, and meropenem, cephalosporins such as cefadroxil, cefazolin, cephalexin, cefaclor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, and cefepime, glycopeptides such as teicoplanin and vancomycin, macrolides such as azithromycin, clarithromycin, dirithromycin, erythromycin, roxithromycin, and troleandomycin, monobactams such as aztreonam, penicillins such as amoxicillin, ampicillin, azlocillin, carbenicillin, cloxacillin, dicloxacillin, flucloxacillin, mezlocillin, nafcillin, penicillin, piperacillin, and ticarcillin, polypeptides such as bacitracin, colistin, and polymyxin B, quinolones such as ciprofloxacin, enoxacin, gatifloxacin, levofloxacin, lomefloxacin, moxifloxacin, norfloxacin, ofloxacin, and trovafloxacin, sulfonamides such as mafenide, prontosil, sulfacetamide, sulfamethizole, sulfanilamide, sulfasalazine, sulfisoxazole, trimethoprim, and trimethoprim-sulfamethoxazole (Co-trimoxazole), tetracyclines such as demeclocycline, doxycycline, minocycline, oxytetracycline, and tetracycline, chloramphenicol, clindamycin, ethambutol, fosfomycin, furazolidone, isoniazid, linezolid, metronidazole, mupirocin, nitrofurantoin, platensimycin, pyrazinamide, quinupristin/dalfopristin, rifampin, and spectinomycin.

Further pharmaceutically active ingredients that can be used in combination with the compound of formula (I) are antifungal drugs. They are, but are not limited to polyene antimycotic such as natamycin, rimocidin, filipin, nystatin, and amphotericin B, imidazoles and triazoles such as miconazole, ketoconazole, clotrimazole, econazole, bifonazole, butoconazole, fenticonazole, isoconazole, oxiconazole, sertaconazole, sulconazole, tioconazole, fluconazole, itraconazole, ravuconazole, posaconazole, voriconazole, and terconazole, allylamines such as terbinafine, amorolfine, naftifine, and butenafine, echinocandins such as anidulafungin, caspofungin, and micafungin, flucytosine, griseofulvin, gentian violet, ciclopirox, tolnaftate, undecylenic acid, and tea tree oil (melaleuca oil).

Further pharmaceutically active ingredients that can be used in combination with the compound of formula (I) are anti-inflammatory drugs. The anti-inflammatory agents that may be used are, but are not limited to disease-modifying antirheumatic drugs (DMARDs) such as adalimumab, azathioprine, chloroquine, hydroxychloroquine, ciclosporin (Cyclosporine A), D-penicillamine, etanercept, gold salts such as sodium aurothiomalate and auranofin, infliximab, leflunomide, methotrexate (MTX), minocycline, and sulfasalazine (SSZ), histamine receptor antagonists such as doxepin, diphenylhydramine, pyrilamine, chlorpheniramine, cetirizine, promethazine, cyproheptadine, phenindamine, terfenadine, cimetidine, ranitidine, famotidine, thioperamide, clobenpropit, impromidine, and antazoline, bradykinin and kallidin receptor antagonists such as des-Arg⁹-[Leu⁸]-bradykinin, HOE140, CP0127, FR173657, and FR190997, nonsteroidal anti-inflammatory drugs (NSAIDs) such as salicylates, e.g. aspirin, diflunisal, acetaminophen, indomethacin, sulindac, etodolac, fermanates, mefenamic acid, meclofenamate, flufenamic acid, tolmetin, ketorolac, diclofenac, ibuprofen, naproxen, fenoprofen, ketoprofen, flurbiprofen, oxaprozin, piroxicam, meloxicam, nabumetone, celecoxib, valdecobix, parecobix, etoricobix, lumaricobix, rofecobix, apazone, nimesulide, colchicines, allopurinol, probenecid, and benzbromarone, β₂ adrenergic receptor agonists such as albuterol, levalbuterol, metaproterenol, terbutaline, pirbuterol, salmeterol xinafoate, formoterol, clenbuterol, fenoterol, reproterol, and salbutamol, glucocorticoids such as alclometasone dipropionate, amcinonide, beclomethasone dipropionate, betamethasone, betamethasone dipropionate, betamethasonesodium phosphate, betamethasonesodium phosphate and acetate, betamethasone valerate, budesonide, ciclesonide, clobetasol propionate, clocortolone pivalate, cortisol, cortisol acetate, cortisol butyrate, cortisol cypionate, cortisol sodium phosphate, cortisol sodium succinate, mometasone furoate, prednisolone, prednisolone acetate, prednisolone sodium phosphate, prednisolone tebutate, cortisol valerate, cortisone acetate, desonide, desoximetasone, dexamethasone, dexamethasone acetate, dexamethasone sodium phosphate, diflorasone diacetate, fludrocortisone acetate, flunisolide, fluocinolone acetonide, fluocinonide, fluorometholone, fluorometholone acetate, flurandrenolide, halcinonide, medrysone, methylprednisolone, methylprednisolone acetate, methylprednisolone sodium succinate, prednisone, triamcinolone, triamincinolone acetonide, triamincinolone diacetate, and triamincinolone hexacetonide, leukotriene receptor antagonists and leukotriene synthesis inhibitors such as zafirlukast, montelukast, and zileuton, inhibitors of phosphodiesterases (PDE) such as xanthine, caffeine, theophylline, theobromine, cilomilast, and roflumilast, immunosuppreesive agents such as azathioprine, 6-mercaptopurine, infliximab, tetracycline, ciclosporin A, tacrolimus, pimecrolimus, sirolimus, methotrexate, azathioprine, and mycophenolatmofetile, muscarinic receptor antagonists such as tiotropiumbromide, ipratropiumbromide, and oxitropiumbromide, active pharmaceutical ingredients reducing the release of inflammatory mediators such as nedocromil, ketotifen, lodoxamide-trometamol, and cromoglicin acid, vitamin D receptor agonists such as calcipotriol and talcalcitol, vitamin A acid receptor agonists (RARα) such as tretinoin, isotretionin, acitretin, tazaroten, adapalen, and bexaroten, RXR receptor agonists such as bexaroten, active pharmaceutical ingredients for the treatment of psoriasis such as dithranol and methoxsalen, anti-IgE therapy with omalizumab, compounds with antioxidative activity such as ascorbic acid, gallic acid and derivates thereof, catechins, tocopherol-derived compounds, BHA, BHT, EDTA, tert-butylhydroquinone, citric acid, and acetic acid.

Further pharmaceutically active ingredients that can be used in combination with the compound of formula (I) are neurologically active drugs. The neurologically active agents that may be used are, but are not limited to antidepressants such as monoamine oxidase inhibitors (MAOI), including harmaline, iproniazid, isocarboxazid, moclobemide, nialamide, pargyline, phenelzine, selegiline, toloxatone, tranylcypromine, rasagiline, reversible inhibitors of monoamine oxidase A (RIMA) such as brofaromine, moclobemide, dopamine re-uptake inhibitors (DARI) such as amineptine, phenmetrazine, vanoxerine, norepinephrine-dopamine re-uptake inhibitors such as bupropion, norepinephrine re-uptake inhibitors (NARI) such as atomoxetine, maprotiline, reboxetine, and viloxazine, serotonin-norepinephrine re-uptake inhibitor (SNRI) such as duloxetine, milnacipran, nefazodone, and venlafaxine, selective serotonin re-uptake inhibitors (SSRI) such as alaproclate, citalopram, dapoxetine, escitalopram, etoperidone, fluoxetine, fluvoxamine, paroxetine, sertraline, and zimelidine, selective serotonin re-uptake enhancer (SSRE) such as tianeptine, tricyclic antidepressants (TCA) such as amitriptyline, amoxapine, butriptyline, clomipramine, desipramine, dibenzepin, dothiepin, doxepin, imipramine, iprindole, lofepramine, melitracen, nortriptyline, opipramol, protriptyline, and trimipramine, tetracyclic antidepressants such as maprotiline, mianserin, nefazodone, and trazodone, NK1 receptor antagonists such as aprepitant, noradrenergic and specific serotonergic antidepressant (NaSSA) such as mirtazapine and mianserine, anxiolytics, such as benzodiazepines such as clobazam, clonazepam, clorazepate, midazolam, lorazepam, alprazolam, and diazepam, non-benzodiazepines such as the serotonin 1A agonist buspirone, barbiturates (e.g. amobarbital, butalbital, cyclobarbital, pentobarbital, allobarbital, methylphenobarbital, phenobarbital, secbutabarbital, and vinylbita), meprobamate, carisoprodol, and tybamate, antipsychotics such as phenothiazinessuch as chlorpromazine, fluphenazine, perphenazine, prochlorperazine, thioridazine, and trifluoperazine, butyrophenones such as haloperidol, droperidol, and pimozide, atypical antipsychotics such as clozapine, olanzapine, risperidone, quetiapine, ziprasidone, amisulpride, melperone, paliperidone, symbyax, partial dopamine antagonists, including aripiprazole, bifeprunox, and norclozapine, active pahramaceutical ingredients for the treatment of Parkinson such as dopa decarboxylase inhibitors, includining carbidopa/levodopa (co-careldopa), benserazide/levodopa (co-beneldopa), duodopa (combination of levodopa and carbidopa) also in combination with COMT inhibitors including tolcapone and entacapone, dopamine-agonists, including bromocriptine, pergolide, pramipexole, ropinirole, cabergoline, apomorphine, and lisuride, monoamine oxidase-B (MAO-B) inhibitors, including selegiline and rasagiline, active pahramaceutical ingredients for the treatment of Alzheimer/Dementia such as acetylcholinesterase (AChE) inhibitors, including donezepil, galantamin, rivastigmin, metrifonate, physostigmine, eostigmine, pyridostigmine, ambenonium, and demarcarium, tacrine and edrophonium, extracts of Gingko biloba leaves, NMDA (N-methyl-D-aspartic acid) receptor antagonists, including memantine, amantadine, dextromethorphan, dextrorphan, ibogaine, ketamine, nitrous oxide, and phencyclidine, vaccines training the immune system to recognize and attack beta-amyloid, active pahramaceutical ingredients for the treatment of multiple sclerosis such as intravenous corticosteroids, Interferon beta-1a or beta-1b, glatiramer acetate, mitoxantrone, natalizumab, and inosine, chemotherapy drugs, including azathioprine, cladribine, mitoxantrone, cyclophosphamide, and methotrexate, sphingosine-1-phosphate receptor modulators such as fingolimod, analgenics such as non-steroidal anti-inflammatory drugs (NSAID) including salicylates (including aspirin, amoxiprin, benorilate, choline magnesium salicylate, diflunisal, faislamine, methyl salicylate, magnesium salicylate, salicyl salicylate (salsalate), arylalkanoic acids (including diclofenac, ceclofenac, acemetacin, bromfenac, etodolac, indometacin, ketorolac, nabumetone, sulindac, tolmetin), 2-arylpropionic acids (including ibuprofen, carprofen, fenbufen, fenoprofen, flurbiprofen, ketoprofen, loxoprofen, naproxen, tiaprofenic acid, suprofen), N-arylanthranilic acids (including mefenamic acid and meclofenamic acid), pyrazolidine derivatives (including phenylbutazone, azapropazone, metamizole, oxyphenbutazone), oxicams (including piroxicam, lornoxicam, meloxicam, tenoxicam), coxibs (including celecoxib, etoricoxib, lumiracoxib, parecoxib, rofecoxib, valdecoxib), sulphonanilides (including nimesulide), licofelone and omega-3 fatty acids, paracetamol, opioid receptor agonists such as phenanthrenes naturally occurring in opium (morphine, codeine, thebaine), preparations of mixed opium alkaloids, including papaveretum, semisynthetic and synthetic derivatives of opium alkaloids including diacetylmorphine (heroin), oxycodone, hydrocodone, dihydrocodeine, hydromorphone, oxymorphone, nicomorphine, fentanyl, alfentanil, sufentanil, remifentanil, carfentanyl, ohmefentanyl, pethidine (meperidine), ketobemidone, MPPP (1-methyl 4-phenyl 4-propionoxypiperidine), allylprodine, prodine, PEPAP (1-methyl-4-phenyl-4-propionoxypiperidine), diphenylpropylamine derivatives such as propoxyphene, dextropropoxyphene, dextromoramide, bezitramide, piritramide, methadone, levo-alphacetylmethadol (LAAM), loperamide, diphenoxylate, benzomorphane derivatives such as pentazocine and phenazocine, oripavine derivatives such as buprenorphine and etorphine, morphinane derivatives such as butorphanol, nalbufine, levorphanol, levomethorphan, and atypical opiods including dezocine, lefetamine, tilidine, and tramadol, active pahramaceutical ingredients for the treatment of migraine tryptamin drugs (tryptans) that agonize serotonine receptors such as sumatriptan, rizatriptan, naratriptan, zolmitriptan, eletriptan, almotriptan, and frovatriptan, diphenhydramine hydrochloride, ergot alkaloids, derivates and synthic ergolines such as ergotamine, dihydroergotamine, and methysergide, beta-adrenergic antagonists such as dichloroisoprenaline, practolol, pronethaolol, alprenolol, carteolol, levobunolol, mepindolol, metipranolol, nadolol, oxprenolol, penbutolol, and pindolol, propranolol, sotalol, timolol, acebutolol, atenolol, betaxolol, bisoprolol, esmolol, metoprolol, nebivolol, carvedilol, celiprolol, labetalol, and butoxamine, anticonvultans such as valproic acid, sodium valproate, divalproex sodium, topiramate, felbamate, vigabatrin, progabide, tiagabine, gabapentin, pregabalin, ethotoin, phenytoin, mephenytoin, fosphenytoin, paramethadione, trimethadione, ethadione, beclamide, primidone, brivaracetam, levetiracetam, seletracetam, ethosuximide, phensuximide, mesuximide, acetazolamide, sulthiame, methazolamide, zonisamide, lamotrigine, pheneturide, phenacemide, valpromide, and valnoctamide.

Further pharmaceutically active ingredients that can be used in combination with the compound of formula (I) are prostaglandins and analogues thereof, for instance latanoprost.

Further pharmaceutically active ingredients that can be used in combination with the compound of formula (I) are angiogenic-related and hyperprolifertive disorders including drugs for the treatment of cancers, tumors, metastasis, and neoplasms. The active pharmaceutical ingredients that may be used are, but are not limited to cytostatic drugs such as alkylating agents (e.g. busulfan and treosulfan), ethylenimines and methylmelamines (e.g. thiotepa and altretamine), nitrosoureas (e.g. carmustine and streptozocin), nitrogen mustards (e.g. mechlorethamine, chlorambucil, cyclophosphamide, ifosfamide, and melphalan), antimetabolites such as folic acid analogs (e.g. methotrexate, pemetrexed, and raltitrexed), purine analogs and related inhibitors (e.g. cladribine, clofarabine, fludarabine, mercaptopurine, nelarabine, pentostatin, and thioguanine), pyrimidine analogs (e.g. azacitidine, capecitabine, cytarabine, decitabine, fluorouracil, gemcitabine, and tegafur), plant alkaloids such as epipodophyllotoxins (e.g. etoposid and etoposide phosphate), taxanes (e.g. docetaxel and paclitaxel), vinca alkaloids (e.g. vinblastine, vincristine, vindesine, and vinorelbine), antibiotics such as anthracyclines (e.g. daunorubicin, doxorubicin, epirubicin, idarubicin, and mitoxantron), bleomycin, dactinomycin, mitomycin, platinum coordination complexes (e.g. carboplatin, cisplatin, and oxaliplatin), campthotecins (e.g. irinotecan and topotecan), all-trans-retinoic acid, amsacrine, anagrelide, L-asparaginase, dacarbazine, estramustine phosphate, hydroxycarbamide, miltefosine, procarbazine, temozolomide, hormones such as anti-androgens (e.g. bicalutamide, cyproterone acetate, and flutamide), antiestrogens (e.g. fulvestrant, tamoxifen, toremifene, raloxifen, and clomifen), aromatase inhibitors (e.g. anastrozole, exemestane, and letrozole), gestagens (e.g. medroxyprogesterone and megestrol), gonadotropin-releasing hormone analogs (e.g. buserelin, goserelin, leuprorelin, and triptorelin), estrogens (e.g. fosfestrol, polyestradiol, dethylstilbestrol, and ethinylestradiol), octreotide, cytokines such as growth factors (e.g. aldesleukin, darbepoetin, erythropoetin, filgrastim (G-CSF), pegfilgrastim, lenograstim (G-CSF), oprelvekin, and sagramostim), interferones (e.g. alpha-interferon and beta-interferon), monoclonal antibodies (e.g. alemtuzumab, bevacizumab, cetuximab, gemtuzumab, rituximab, trastuzumab, ⁹⁰Y-ibritumomab, and ¹³¹I-tositumomab), signal transduction inhibitors and various other drugs (e.g. atrasentan, bexarotene, bortezomib, dasatinib, erlotinib, gefitinib, imatinib, lapatinib, lenalidomid, nilotinib, sorafenib, sunitinib, thalidomid, and angiostatin), antiemetics (e.g. aprepitant, dolasetron, granisetron, ondansetron, palonosetron, and tropisetron), supportive compounds (e.g. amifostine, carboxypeptidase, folinic acid, and rasburicase), bisphosphonates (e.g. clodronate, ibandronate, pamidronate, and zoledronate), P-glycoprotein (P-gp) inhibitors (e.g. verapramil).

Further pharmaceutically active ingredients that can be used in combination with the compound of formula (I) are drug affecting renal and cardiovascular function. The active pharmaceutical ingredients that may be used are, but are not limited to diuretics such as inhibitors of carbonic anhydrase (e.g. acetazolamide, dichlorphenamide, and methazolamide), osmotic diuretics (e.g. glycerine, isorbide, mannitol, and urea), inhibitors of Na⁺-K⁺-2C1-symport (e.g. furosemide, bumetanide, ethacrynic acid, torsemide, axosemide, piretanide, and tripamide), inhibitors of Na⁺-Cl⁻ symport (e.g. bendroflumethazide, chlorothazide, hydroflumethazide, methylcyclothiazide, polythiazide, chlorthalidone, indapamide, metolazone, and quinethazone), inhibitors of renal epithelial sodium channels (e.g. mailoride and triamterene), antagonists of mineralocorticoid receptors (e.g. pironolactone, canrenone, potassium canrenoate, and eplerenone), vasopressin and agents affecting the renal conservation of water including Vasopressin receptor agonists such as naturally occuring vasopressin-like peptides (e.g. arginine vasopressin, lypressin, phenypressin, vasotocin, arginine conopressin, lysine conopressin, and lucost subesophgeal ganglia peptide), synthetic vasopressin peptides (e.g. desmopressin), nonpeptide agonists, vasopressin receptor antagonists such as peptide antagonists, nonpeptide antagonsists, drugs interrupting the renin-angiotensin system such as angiotensin-converting enzyme inhibitors (e.g. benazepril, captopril, enelapril, enalaprilat, fosinopril, lisinopril, moexopril, perindopril, quinapril, ramipril, and trandolapril), angiotensin II-receptor antagonists (e.g. candesartan, irbesartan, losartan, olmesaratan, telmisartan, valsartan, eprosartan), drugs for the treatment of angina such as organic nitrates (e.g. nitroglycerin, isorbide dinitrate, isorbide-5-mononitrate), Ca²⁺ channel blockers (e.g. amlopidine, felodipine, isradipine, nicardipine, nifedipine, diltiazem, verapamil), sympatholytic drugs such as β-adrenergic antagonists (e.g. metoprolol and atenolol), α-adrenergic antagonists (e.g. prozosin, terazosin, doxazosin, phenoxybenzamine, phentolamine), mixed adrenergic antagonists (e.g. labetalol, carvadilol), centrally acting agents (e.g. methyldopa, clonidine, guanabenz, guanfacine), adrenergic neuron blocking agents (e.g. guanadrel and reserpine), antiarrhytmic drugs (e.g. adenosine, amiodarone, digitoxin, diltiazem, disopyramide, dofetilide, esmolol, flecainide, ibutilide, lidocaine, mexiletine, moricizine, procainamide, propafenone, propranolol, quinidine, sotalol, tocainide), drugs for therapy of hypercholesterolemia and dyslipidemia such as statins (e.g. mevastatin, lovastatin, simvastatin, pravastatin, atorvastatin, rosuvastatin), bile acid sequestrants (e.g. cholestyramine and colestipol), peroxisome proliferator activated receptor (PPAR) activators (e.g. clofibrate, gemfibrozil, fenofibrate, ciprofibrate, benzafibrate).

Further pharmaceutically active ingredients that can be used in combination with the compound of formula (I) are drug affecting gastrointestinal and liver function. The active pharmaceutical ingredients that may be used are, but are not limited to drugs for therapy of gastric acidity, peptic ulcers, and gastroesophageal reflux disease such as proton pump inhibitors (e.g. lansoprazole, omeprazole, rabeprazole, pantoprazole), H2-receptor antagonists (e.g. cimetidine, ranitidine, famotidine, nizatidine), agents that enhance mucosal defense (e.g. prostaglandin analogs), sulphated polysaccharides (e.g. sucralfate), antacids, dopamine-receptor antagonists (e.g. metoclopramide, domperidone), serotonin receptor modulators (e.g. alosetron, tegaserod, prucalopride, cisapride), laxatives such as luminally active agents (e.g. hydrophilic colloids, osmotic agents, stool-wetting agents), non-specific stimulants and irritants with effects on fluid secretion and motility (e.g. diphenylmethanes, bisacodyl, anthraquinones), prokinetic agents acting on motility (e.g. 5-HT4 receptor antagonists, opiod receptor antagonists), antidiarrheal agents such as bulk-forming and hydroscopic agents (e.g. carboxymethylcellulose, calcium, polycarbophil, silicates), bile acid sequestrants (e.g. cholestyramine, colestipol, colesevalam), bismuth compounds (e.g. bismuth subsalicylate), antimotility and antisecretory agents such as opiods (e.g. loperamide, diphenoylate, difenoxin, octreotide, somatostatin, berberine), antiemitic agents such as 5-HT3 receptor antagonists (e.g. ondansetron), centrally acting dopamine receptor antagonists (e.g. metoclopramide and promethazine), histamine H1 receptor antagonists (e.g. cyclizine), muscarinic receptor antagonists (e.g. hyoscine), neuokinin receptor antagonists, cannabinoid receptor antagonists (e.g. dronabinaol), drugs to treat inflammatory bowel disease such as sulfasalazine and related agents (e.g. mesalamine, sulfapyridine, olsalazine, balsalazide), antibiotics (e.g. metronidazide, ciproflaxcin), corticosteroids, immunomodulators (e.g. methotrexate, cyclosporine), biological response modifiers (e.g. infliximab), bile acids (e.g. cholic acid, chodeoxycholic acid, deoxycholic acid, ursodeoxycholic acid), thiazides, potassium citrate, sodium citrate, magnesium citrate, allopurinol, oxybutynin, tolterodine, trospium chloride, darifenacin, solifenacin, and flavoxate.

Further pharmaceutically active ingredients that can be used in combination with the compound of formula (I) are drug for hematological disorders. The active pharmaceutical ingredients that may be used are, but are not limited to drugs acting on the blood and blood forming organs including hematopoietic agents such as hematopoietic growth factors (e.g. erythropoietin (EPO), granulocyte-macrophage colony stimulating factor (GM-CSF), granulocyte colony-stimulating factor (G-CSF), interleukin-11, and thrombopoietin), drugs effective in iron deficiency and other hypochromic anemias such as iron and iron salts (e.g. ferrous sulphate, ferrous gluconate, polysaccharide-iron complexes, ferrum reductum, iron phosphate salts), pyridoxine, vitamin B12, folic acid, blood coagulation, and anticoagulant, thrombolytic, and antiplatelet drugs such as anticoagulants (e.g. heparin, synthetic heparin derivatives, lepirudin, bivalirudin, argatroban, danaparoid, drotrecogin alfa), oral anticoagulants (e.g. warfarin, phenprocoumon, acenocoumarol, anisindione, phenindione, ximelagatran), and antiplatelet drugs (e.g. aspirin, dipyridamole, ticlopidine, clopidogrel, abciximab, eptifibatide, tirofiban, and vitamin K).

Further pharmaceutically active ingredients that can be used in combination with the compound of formula (I) are drug affecting metabolic function. The active pharmaceutical ingredients that may be used are, but are not limited to hypoglycemic agents such as insulin and insulin analogs (e.g. the preparations lispro, apart, glulisine, lente, ultralente, glargine), sulfonylurea (e.g. tolbutamide, chlorpropamide, tolazamide, acetohexamide, glyburide, glipizide, gliclazide, glimepiride, glibenclamide, gliquidone), repaglinide, nateglinide, biguanides, thiazolidinediones (e.g. troglitazone, rosiglitazone, pioglitazone), α-glucosidase inhibitors (e.g. acarbose, miglitol, voglibose), glucagon, somatostatin (e.g. octreotide, lanreotide), and diazoxide.

Further pharmaceutically active ingredients that can be used in combination with the compound of formula (I) are drug affecting urology-related function. The active pharmaceutical ingredients that may be used are, but are not limited to methanamine, nitrofurantion, and phenazopyridine.

The active compounds of these combinations can be administered simultaneously or separately, they can be formulated within one dosage form or they can be in different dosage forms. Both pharmaceutically active ingredients can also be administered in one dosage form followed by a dosage form comprising only one active pharmaceutical ingredient and vice versa. With respect to the timing of the administration of these combinations, depending on the disease to be treated, the severity of the disease and the patient in question, various possibilities can be realized. Both (or several) active compounds can be administered concurrently in either the same dosage form or different dosage forms. Both active compounds can be administered within a short time frame, e.g. one only few seconds after the other, or they can be given with a significant time difference, e.g. several hours, after specific biological conditions are met.

Actual dosage levels of active ingredients in the composition of the present invention may be varied so as to obtain an amount of active ingredient that is effective to obtain the desired therapeutic response for a particular composition and method of administration. The selected dosage level, therefore, depends on the desired therapeutic effect, on the route of administration, on the desired duration of treatment and other factors. It will be understood, however, that the specific dose level for any particular patient will depend upon a variety of factors including the body weight, general health, sex diet, time and route of administration, rates of absorption and excretion, and the severity of the particular disease being treated.

The amount used of compound of formula (I) corresponds to the amount required to obtain the desired result using the pharmaceutical compositions. One skilled in this art is capable of evaluating this effective amount, which depends on the derivative used, the individual on whom it is applied, and the time of this application. To provide an order of magnitude, in the pharmaceutical compositions according to the invention, the compound of formula may be administered in an amount representing from 0.001% to 40% by weight, preferentially 0.005% to 30% by weight and more preferentially from 0.01% to 20% by weight.

The amount used of the further active pharmaceutical ingredient corresponds to the amount required to obtain the desired result using the pharmaceutical compositions. One skilled in this art is capable of evaluating this effective amount, which depends on the derivative used, the individual on whom it is applied, and the time of this application. To provide an order of magnitude, in the pharmaceutical compositions according to the invention, the further active pharmaceutical ingredient may be administered in an amount representing from 0.001 % to 30% by weight, preferentially 0.005% to 20% by weight and more preferentially from 0.01% to 10% by weight.

For the therapy of the above-mentioned conditions, the compound of formula (I) may be used as the compound itself, but it is preferably in the form of a pharmaceutical composition with an acceptable carrier. The carrier must be acceptable in the sense that it is compatible with the other ingredients of the composition and is not harmful for the patient's health. The carrier may be a solid or a liquid or both and is preferably formulated with the compound as a single dose, for example as a tablet, which may contain from 0.05 % to 95 % by weight of the active ingredient. Other pharmaceutically active substances may be present. The pharmaceutical compositions of the invention can be produced by one of the known pharmaceutical methods, which essentially consist of mixing the ingredients with pharmacologically acceptable carriers and/or excipients.

Pharmaceutical compositions of the invention are those suitable for oral, rectal, topical, peroral (for example sublingual) and parenteral (for example subcutaneous, intramuscular, intradermal or intravenous) administration, although the most suitable mode of administration depends in each individual case on the patient to be treated, the nature and severity of the condition to be treated and on the nature of the compound of formula (I) used in each case.

Coated formulations and coated slow-release formulations also belong within the framework of the invention. Preference is given to acid- and gastric juice-resistant formulations. Suitable coatings resistant to gastric juice comprise cellulose acetate phthalate, polyvinyl acetate phthalate, hydroxypropylmethylcellulose phthalate and anionic polymers of methacrylic acid and methyl methacrylate.

Suitable pharmaceutical compounds for oral administration may be in the form of separate units such as, for example, capsules, cachets, suckable tablets or tablets, each of which contain a defined amount of the compound of formula (I); as powders or granules, as solution or suspension in an aqueous or nonaqueous liquid; or as an oil-in-water or water-in-oil emulsion. These compositions may be prepared by any suitable pharmaceutical method which includes a step in which the active ingredient and the carrier (which may consist of one or more additional ingredients) are brought into contact. The compositions are generally produced by uniform and homogeneous mixing of the active ingredient with a liquid and/or finely divided solid carrier, after which the product is shaped, if necessary. Thus, for example, a tablet can be produced by compressing or molding a powder or granules of the compound, where appropriate with one or more additional ingredients. Compressed tablets can be produced by tableting the compound in free-flowing form such as, for example, a powder or granules, where appropriate mixed with a binder, glidant, inert diluent and/or one or more surface-active/dispersing agent(s) in a suitable machine. Molded tablets can be produced by molding the compound, which is in powder form and is moistened with an inert liquid diluent, in a suitable machine.

Pharmaceutical compositions which are suitable for peroral (sublingual) administration comprise suckable tablets which contain a compound of formula (I) with a flavoring, normally sucrose and gum arabic or tragacanth, and pastilles which comprise the compound in an inert base such as gelatin and glycerol or sucrose and gum arabic.

Pharmaceutical compositions suitable for parenteral administration comprise preferably sterile aqueous preparations of a compound of formula (I), which are preferably isotonic with the blood of the intended recipient. These preparations are preferably administered intravenously, although administration may also take place by subcutaneous, intramuscular or intradermal injection. These preparations can preferably be produced by mixing the compound with water and making the resulting solution sterile and isotonic with blood. Injectable compositions of the invention generally contain from 0.1 to 5 % by weight of the active compound.

Pharmaceutical compositions suitable for rectal administration are preferably in the form of single-dose suppositories. These can be produced by mixing a compound of the formula (I) with one or more conventional solid carriers, for example cocoa butter, and shaping the resulting mixture.

Pharmaceutical compositions suitable for topical use on the skin are preferably in the form of ointment, cream, lotion, paste, spray, aerosol or oil. Carriers which can be used are petrolatum, lanolin, polyethylene glycols, alcohols and combinations of two or more of these substances. The active ingredient is generally present in a concentration of from 0.1 to 15 % by weight of the composition, for example from 0.5 to 2 %.

Transdermal administration is also possible. Pharmaceutical compositions suitable for transdermal uses can be in the form of single plasters which are suitable for long-term close contact with the patient's epidermis. Such plasters suitably contain the active ingredient in an aqueous solution which is buffered where appropriate, dissolved and/or dispersed in an adhesive or dispersed in a polymer. A suitable active ingredient concentration is about 1 % to 35 %, preferably about 3 % to 15 %. A particular possibility is for the active ingredient to be released by electrotransport or iontophoresis as described, for example, in Pharmaceutical Research, 2(6): 318 (1986).

The compound of formula (I) may also be administered in combination with other active ingredients. The pharmaceutical compositions may also comprise pharmaceutically tolerable components, which are considered to be inactive pharmaceutical ingredients. The amounts of the various components of the physiological medium of the pharmaceutical composition according to the invention are those generally included in the fields under consideration. When the pharmaceutical composition is an emulsion, the proportion of the fatty phase may range from 2% to 80% by weight and preferably from 5% to 50% by weight relative to the total weight of the pharmaceutical composition.

The aqueous phase is adjusted as a function of the content, in the fatty phase, of compound of formula (I), of alcohol and nonionic surfactant and also that of the optional additional ingredients, to obtain 100% by weight. In practice, the aqueous phase represents from 5% to 90% by weight. The fatty phase may contain fatty or oily compounds, which are liquid at room temperature (25°C.), generally known as oils, waxes and pasty or semi-solid products. These oils may be mutually compatible and may form a macroscopically homogeneous liquid fatty phase. The aqueous phase contains water and optionally a water-miscible ingredient, for instance polyols such as propylene glycol, glycerol or sorbitol.

In particular, the pharmaceutical composition often contains one or more components such as nonionic surfactants. The nonionic surfactant or the mixture of nonionic surfactants with a hydrophilic-lipophilic balance (HLB) of greater than 10 is (are) preferably used in an amount sufficient to dissolve with the compound of formula (I). In practice, this nonionic surfactant or mixture of nonionic surfactants may be included in the compositions of the invention in a concentration ranging from 0.01% to 10% by weight, preferentially from 0.05% to 5% by weight and more preferentially from 0.1 % to 2% by weight.

More specifically, pharmaceutical compositions of the present invention can contain nonionic surfactants having a HLB of greater than 10 [Griffin, W. C., J. Soc. Cosmet. Chem.,1949, 1, 311, and 1954, 5, 249] and which may be up to 20. These are compounds that are well known per se [Handbook of Surfactants by M. R. Porter, published by Blackie & Son (Glasgow and London), 1991, pp. 116-178]. Thus, they may be selected especially from among polyethoxylated, polypropoxylated or polyglycerolated fatty acids, (C1-C20) alkylphenols, alpha-diols and alcohols, which are preferably hydrogenated, with a fatty chain containing, for example, from 8 to 22 carbon atoms, the mean number of ethylene oxide or propylene oxide structural units optionally ranging especially from 3.5 to 200 (for example from 5 to 100) and the number of glycerol groups optionally ranging especially from 2 to 100 (for example from 3 to 50), and mixtures thereof.

Also exemplary are copolymers of ethylene oxide and propylene oxide, condensates of ethylene oxide and propylene oxide on fatty alcohols; polyethoxylated fatty amides and preferably those containing on average from 3.5 to 200 mol of propylene oxide and/or ethylene oxide; polyglycerolated fatty amides and preferably those containing on average from 1.5 to 40; ethoxylated fatty acid esters of sorbitan especially containing from 2 to 30 mol on average of ethylene oxide and a fatty chain especially containing from 8 to 22 (for example from 12 to 18) carbon atoms; fatty acid esters of sucrose; fatty acid esters of polyethylene glycol; (C6-C24)alkylpolyglycosides; N-(C6-C24) alkylglucamine derivatives; amine oxides such as(C10-C14) alkylamine oxides; and mixtures thereof.

In the pharmaceutical compositions, an alcohol may be used alone or as a mixture and is selected from among C1-C4 alcohols such as ethanol or isopropanol, and mixtures thereof. The C1-C4 alcohol is preferably included in an amount sufficient to dissolve with the nonionic surfactant the compound of formula (I). In practice, the C1-C4 alcohol or the mixture of C1-C4 alcohols may be included in the pharmaceutical compositions of the invention in a concentration ranging from 2% to 80% of the total weight of the composition, preferentially from 10% to 70% and more preferentially from 20% to 60% by weight relative to the total weight of the pharmaceutical composition.

If the pharmaceutical compositions are for topical application to the skin, the pharmaceutical composition should contain a non-toxic physiologically acceptable medium that can be applied to human skin. For a topical application to the skin, the pharmaceutical composition may be in the form of a solution, a suspension, an emulsion or a dispersion of more or less fluid consistency and especially liquid or semi-liquid consistency, obtained by dispersing a fatty phase in an aqueous phase (O/W) or, conversely, (W/O), or alternatively a gel. A pharmaceutical composition in the form of a mousse or in the form of a spray or an aerosol then comprising a pressurized propellant may also be provided.

The pharmaceutical compositions of the invention may also comprise one or more other ingredients usually employed in the fields under consideration, selected from among formulation additives, for instance aqueous-phase or oily-phase thickeners or gelling agents, dyestuffs that are soluble in the medium of the pharmaceutical composition, solid particles such as mineral or organic fillers or pigments in the form of microparticles or nanoparticles, preservatives, fragrances, hydrotopes or electrolytes, neutralizers (acidifying or basifying agents), propellants, anionic, cationic or amphoteric surfactants, polymers, in particular watersoluble or water-dispersible anionic, nonionic, cationic or amphoteric film-forming polymers, mineral or organic salts, chelating agents; mixtures thereof.

These additives may be present in the pharmaceutical composition in the amounts generally employed in pharmaceutical, and especially in a proportion of from 0.01% to 50% and preferably from 0.1% to 20%, for example from 0.1 % to 10%, of the total weight of the pharmaceutical composition. Oils that may be included according to the invention may be made of oils of mineral origin (liquid petroleum jelly or hydrogenated isoparaffin), oils of plant origin (liquid fraction of shea butter, sunflower oil, apricot oil, soybean oil, fatty alcohol or fatty acid), oils of animal origin (perhydrosqualene), synthetic oils (fatty acid esters or purcellin oil), silicone oils (linear or cyclic polydimethylsiloxanes, and phenyl trimethicones) and fluoro oils (perfluoropolyethers).

Waxes that may be mentioned include silicone waxes, beeswax, candelilla wax, rice bran wax, carnauba wax, paraffin wax or polyethylene wax. When the pharmaceutical composition is in emulsion form, it also contains one or more emulsifiers and optionally one or more co-emulsifiers generally employed in pharmaceuticals. Their nature also depends on the sense of the emulsion. In practice, the emulsifier and, optionally, the co-emulsifier are present in the pharmaceutical composition in a proportion ranging from 0.1% to 30% by weight, preferably from 0.5% to 20% by weight and better still from 1% to 8% by weight. The emulsion may also contain lipid vesicles and especially liposomes. As thickeners or gelling agents that may be included according to the invention, mention may be made of thickening or gelling polymers, for instance crosslinked polyacrylic acids, associative polymers and non-polymeric thickeners or gelling agents, for instance modified or unmodified clays, amides and metal salts of fatty acids.

Further inactive pharmaceutical ingredients or carriers can also be a medically appropriate nano-particle, dendrimer, liposome, microbubble or polyethylene glycol (PEG). The pharmaceutical compositions of the present invention may include additional active ingredients like physiologically acceptable carriers, adjuvants or vehicles, which are collectively referred to herein as carriers, for parenteral injection, for oral administration in solid or liquid form, for rectal topical administration and the like.

Compositions suitable for parenteral injection may comprise physiologically acceptable sterile aqueous or nonaqueous solutions, stabilizers, antioxidants, preservatives (e.g. ascorbic acid, sodium sulfite, sodium hydrogene sulfite, benzyl alcohol, EDTA), dispersions, suspensions or emulsions and sterile powders for reconstitution into sterile injectable solution or dispersion. Examples of suitable aqueous and nonaqueous carriers, diluents, solvents or vehicles include water, ethanol, polyol, (propylene glycol, polyethylene glycol, glycerol and the like), suitable mixtures thereof, vegetable oils (such as olive or canola oil) and injectable organic esters such as ethyl oleate. Proper fluidity can be maintained, for examples, by the use of a coating such as lecithin, by the maintenance of the required particle size in the case of dispersions and by the use of surfactants.

These compositions may also contain adjuvants such as preserving, wetting, emulsifying, and dispersing agents. Prevention of the actions of microorganisms can be ensured by various antibacterial and antifungal agents, for examples, parabens, chlorobutanol, phenol, sorbic acid, and the like. It may also be desirable to include isotonic agents, for examples sugars, sodium chloride and the like. Prolonged absorption of the injectable pharmaceutical form can be brought about by the use of agents delaying absorption, for examples aluminium monostearate and gelatin.

If desired, and for more effective distribution, the compounds can be incorporated into slow or timed release or targeted delivery systems such as polymer matrices, liposomes, and microspheres. They may be sterilized, for example, by filtration through a bacteria-retaining filter, or by incorporating sterilizing agents in the form of sterile water, or some other sterile injectable medium immediately before use.

Solid dosage forms for oral administration include capsules, tablets, pills, powders and granules. In such solid dosage forms, compound of formula (I) and the active pharmaceutical ingredient is admixed with at least one inert customary excipient (or carrier) such as sodium citrate or dicalcium phosphate or (i) fillers or extenders, as for example, starches, lactose, sucrose, glucose, mannitol and silicic acid, (ii) binders, as for example, carboxymethylcellulose, alginates, gelatine, polyvinylpyrrolidone, sucrose and acacia, (iii) humectants, as for example, glycerol, (div disintegrating agents, as for example, agar-agar, calcium carbonate, potato or tapioca starch, aliginic acid, certain complex silicates and sodium carbonate, (v) solution retarders, as for examples, paraffin, (vi) absorption accelerators, as for example, quaternary ammonium compounds, (vii) wetting agents, as for examples, cetyl alcohol and glycerol monostearate, (viii) adsorbents, as for example, kaolin and bentonite, and (ix) lubricants, as for example, talc, calcium stearate, magnesium stearate, solid polyethylene glycols, sodium lauryl sulfate and mixtures thereof. In the case of capsules, tablets, and pills, the dosage forms may also comprise buffering agents.

Solid compositions of a similar type may also be employed as fillers in soft and hard-filled gelatine capsules using excipients as lactose or milk sugars as well as high molecular polyethylene glycols and the like. Solid dosage forms such as tablets, dragées, capsules, pills and granules can be prepared with coatings and shells, such as enteric coatings and others well known in the art. They may contain opacifying agents, and can also be of such compositions that they release the active compound or compounds in a certain part of the intestinal tract in a delayed manner. Examples of embedding compositions that can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, cannola oil, caster oil and sesame seed oil, glycerol, tetrahydrofurfuryl alcohol, polyethylene glycols and fatty acid esters of sorbitan or mixtures of these substances, and the like. Besides such inert diluents, the compositions can also include adjuvants, such as wetting agents, emulsifying and suspending agents, sweeting, flavouring and perfuming agents.

Suspensions, in addition to the active compounds, may contain suspending agents, for example, ethoxylated isostearyl alcohols, polyoxyethylene sorbitol and sorbitan esters, microcrystalline cellulose, aluminium metahydroxide, bentonite, agar-agar, tragacanth or mixtures of these substances and the like.

Compositions for rectal administrations are preferably suppositories, which can be prepared by mixing compound of formula (I) and at least one further active pharmaceutical ingredient with suitable nonirritating excipients or carriers such as cacao butter, polyethylene glycol or a suppository wax, which are solid at ordinary temperatures but liquid at body temperature and therefore melt in the rectal or vaginal cavity and release the active component. Dosage forms for topical administration of compound of formula (I), a salt of compound of formula (I) or an isomeric or polymorphic form thereof include ointments, gels, micro emulsions and lotions.

The active components are admixed under sterile conditions with a physiologically acceptable carrier and any needed preservatives, buffers or propellants as may be required. Ophthalmic formulations, eye ointments, suspensions, powder and solutions are also contemplated as being within the scope of this invention.

The active compounds can also be incorporated into or connected to liposomes or administrated in the form of liposomes. As is known in the art, liposomes are generally derived from phospholipids or other lipid substances. Liposomes are formed by mono or multilamellar hydrated liquid crystals that are dispersed in an aqueous medium. Any non-toxic, physiologically acceptable metabolized lipid capable of forming liposomes can be used.

The present compositions in liposome form can contain stabilizers, preservatives, excipients and the like. The preferred lipids are the phospholipids and the phosphatidyl cholines (lecithins), both natural and synthetic. Methods to form liposomes are well known in the art.

Non-parenteral dosage forms may also contain a bioavailability enhancing agent (e.g. enzyme modulateors, antioxidants) appropriate for the protection of the compounds against degradation.

The compound of formula (I) can also be applied by using all types of drug delivery devices. The term "drug delivery device" according to the instant invention means a single-dose or multi-dose, disposable or re-useable device designed to dispense a selected dose of a medicinal product. Said device (e.g. injector, inhaler, nebulizer, Transdermal Therapeutic System, pump) may be of any shape, e.g. compact or pentype. Dose delivery may be provided through a mechanical, optionally manual, or electrical drive mechanism or stored energy drive mechanism or stored energy drive mechanism, such as a spring. Dose selection may be provided through a manual mechanism or electronic mechanism. Additionally, said device may contain components designed to monitor physiological properties such as blood glucose levels. Furthermore, the device may comprise a needle or may be needle-free. In particular, the term "drug delivery device" shall mean a disposable multi-dose pen-type device or a metered dose inhaler having mechanical and manual dose delivery and dose selection mechanisms, which is designed for regular use by persons without formal medical training such as patients. Preferably, the drug delivery device is of the injector-type.

A further aspect of the invention deals with a process for the preparation of a pharmaceutical composition by mixing a compound of formula (I) and at least one further pharmaceutically active ingredient.

In particular, a process for the preparation of a pharmaceutical composition by mixing a compound of formula (I) and at least one further pharmaceutically active ingredient, wherein the composition includes from 0.01% to 20% by weight of compound of formula (I), and 0.01 % to 10% by weight of at least one further active pharmaceutical ingredient based on the total weight of the composition is also subject of this invention.

The pharmaceutical compositions contain, for example, compound of formula (I), at least one further active pharmaceutical ingredient, and inactive pharmaceutical ingredients. The pharmaceutical preparations can be manufactured using standard technologies, known to the person skilled in the art. For this, the compound of formula (I) according to the invention is brought into a suitable dosage form together with one or more pharmaceutical vehicles and/or inactive ingredients and at least one or more other active pharmaceutical ingredients having therapeutic, or prophylactic action, which can then be used as pharmaceutical product.

Other features of the invention will become apparent in the course of the following descriptions of the exemplary embodiments, which are given for illustration of the invention and are not intended to be limiting thereof.

### Examples

In the following examples the percentages are given by weight. The term "qs 100%" means that a sufficient amount of that ingredient is present to make the sum of the amounts for all ingredients equal 100%.

### Example 1

A human clinical trial (trial) was conducted to investigate effects of the disodium salt of compound of formula (I) (1,6-Bis [3-(3-carboxymethylphenyl)-4-(2-α-D-mannopyranosyloxy)-phenyl] hexan) on patients with chronic obstructive pulmonary disease (COPD). The trial was carried out according to known methods and according to the "good clinical practices" (GCP), applicable regulatory requirements, and was based on written approval of the Independent Ethics Committee (IEC). Patients were enrolled in the trial based on inclusion and exclusion criteria known.

Patients were treated by inhalation of the disodium salt of the compound of formula (I) for 10 days. The unit dose strength for nebulization was 70 mg in 4 ml of 0.9% NaCl twice a day (bid). To investigate laboratory parameters the procedure of induced sputum (IS) was used to harvest cells and sputum supernatant for further analysis.

To investigate levels of Interleukin 8 (IL-8) in the sputum supernatant, sputum was frozen after collection. Once, all samples were collected, levels of IL-8 were analyzed by using a commercial available enzyme immunoassay for the in vitro quantification of human IL-8 in biological fluids. IL-8 sample concentrations were determined employing calibration curves.

### Results :

Statistic analysis of the generated data of levels of IL8 in supernatant by Mann-Whitney Rank Sum Test revealed a median value of 15419 pg/ml before and a median value of 4985 pg/ml after treatment with the compound of formula (I). This corresponds to a change of -68% of the IL-8 medians. The analyzed difference in IL-8 levels between before and after treatment is statistically significant (p = 0.031) and clinically relevant.

### Example 2

650 g of an antiviral cream for the treatment viral infections caused by herpes simplex having the following composition is prepared according to known methods by mixing of
5% of acyclovir,
1,3% of the compound of formula (I),
8% of cetostearyl alcohol,
2% of mineral oil,
1.5% ofpoloxamer 407,
3.5% ofpropylene glycol,
0.75% of sodium lauryl sulfate, 2.5% of white petrolatum,
0.15% of preserving agent, and
qs100% of demineralized water.

This composition containing compound (I) and an anti-viral component can be used on the skin to treat herpes simplex infections. A cream comprising a combination of 5% acyclovir and 1.3% of the compound of formula (I), can be applied to affected skin areas 5 times daily for several days. Time to lesion healing and cessation of pain can be monitored.

### Example 3

550 g of a microemulsion having the following composition is prepared according to the methods known to the person skilled in the art by mixing:
1.0% of the compound of formula (I),
12% of Synperonic ® PE/L101 (Poloxamer 331, surfactant, Uniqema),
8% of Tagat ® 02 V (surfactant, Goldschmidt GmbH),
5% of glyceryl triacetate, and
qs100% of propylene glycol/ 0.005 N hydrochloric acid (2:1).

The pH value of the formulation is adjusted to 4.0. This composition can be used for the treatment of infections.

The topical application of 0.05% betamethasone dipropionate cream can be applied to affected skin areas twice daily for 2 weeks. Starting at the first day of betamethasone dipropionat application, affected areas can additionally be treated twice daily with 1% microemulsion of the compound of formula (I) or placebo for 16 weeks. Disease manifestation can be regularly documented by the Psoriasis Area and Severity Index (PASI).

## Claims

1. Use of a compound of formula (I), a salt of this compound or a stereoisomeric or polymorphic form thereof 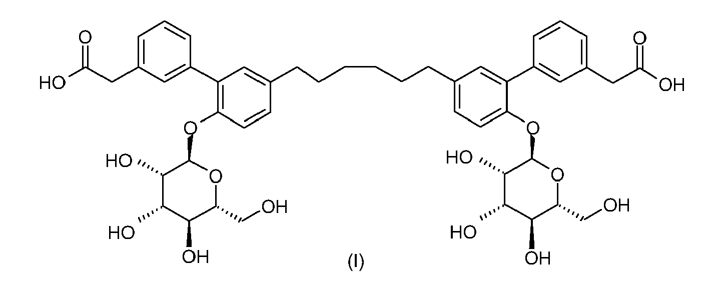 for the preparation of a pharmaceutical composition for the treatment, diagnosis or prophylaxis of Interleukin-8 mediated disorders

2. Use according to claim 1 **characterized in that** the pharmaceutical composition is used for the treatment, diagnosis, or prophylaxis of viral infections.

3. Use according to on of the claims 1 or 2 **characterized in that** the pharmaceutical composition is used for the treatment, diagnosis, or prophylaxis of viral infections caused by cytomegalovirus (CMV), encephalomyocarditis virus (EMCV), poliovirus, influenca virus, parainfluenza respiratory influenza virus, respiratory syncytial virus, Japanese encephalitis virus, Dengue virus, hepatitis A virus (HAV), hepatitis B virus (HBV), hepatitis C virus (HCV), hepatitis D virus (HDV), hepatitis E virus (HEV), hepatitis F virus (HFV), hepatitis G virus (HGV), Epstein Barr Virus (EBV), human immunodeficiency virus type 1 (HIV-1), human immunodeficiency virus type 2 (HIV-2), varicella zoster virus, herpes simplex virus, herpes simplex virus type 1 (HSV-1), herpes simplex virus type 2 (HSV-2), human herpes virus 1, 2, 3, 4, 7, or 8, Kaposi's sarcoma-associated herpesvirus (KSHV), rotavirus, papilloma virus, human papilloma virus (HPV), HPV 1, 2, 3, 4, 5, 8, 9, 11, 12, 13, 14, 15, 16, 17, 18, 19-29, 31, 32, 34, 36-38, 46-50, 56, or 58.

4. Use according to one of the claims 1, 2, or 3 **characterized in that** the pharmaceutical composition is used for the treatment, diagnosis or prophylaxis of neurological disorders.

5. Use according to one of the claims 1, 2, 3, or 4 **characterized in that** the pharmaceutical composition is used for the treatment, diagnosis or prophylaxis of pain.

6. Use according to one of the claims 1, 2, 3, or 4 **characterized in that** the pharmaceutical composition is used for the treatment, diagnosis or prophylaxis of dementia.

7. Use according to claim 1 **characterized in that** the pharmaceutical composition is used for the treatment, diagnosis, or prophylaxis of cardiovascular disorders, urology-related disorders, hematological disorders, metabolic disorders, angiogenetic-related disorders, hyperprolifertive disorders and gastrointestinal and liver disorders.

8. Pharmaceutical compositions comprising the compound of formula (I) and/or a salt of compound of formula (I) and/or a stereoisomeric or polymorphic form thereof in combination with at least one further pharmaceutically active ingredient.

9. Process for the preparation of a pharmaceutical composition by mixing a compound of formula (I) and/or a salt of compound of formula (I) and/or a stereoisomeric or polymorphic form thereof and at least one further pharmaceutically active ingredient.

10. Process for the preparation of a pharmaceutical composition according to claim 9 by mixing a compound of formula (I) and/or a salt of compound of formula (I) and/or a stereoisomeric or polymorphic form thereof and at least one further pharmaceutically active ingredient, wherein the composition comprises from 0.01 % to 20% by weight of compound of formula (I) and/or a salt of compound of formula (I) and/or a stereoisomeric or polymorphic form thereof, and from 0.01 % to 10% by weight of at least one further pharmaceutically active ingredient based on the total weight of the composition.
